# EUROPEAN PATENT APPLICATION

(11) **EP 4 623 845 A1**
(43) Date of publication of application: **01.10.2025**
(21) Application number: 24774718.1
(22) Date of filing: 08.03.2024
(51) Int. Cl.: A61B 17/34

(54) **LYMPHANGIOGENESIS INDUCTION DEVICE**

(30) Priority: 20.03.2023 JP 2023043801
(71) Applicant: Terumo Kabushiki Kaisha, Tokyo 151-0072 (JP)
(72) Inventor: KAWASAKI, Manami, Ashigarakami-gun, Kanagawa 259-0151 (JP); ISHIDA, Masahiro, Ashigarakami-gun, Kanagawa 259-0151 (JP)
(74) Representative: KIPA AB
(86) International application number: PCT/JP2024/008922
(87) International publication number: WO 2024/195579

(57) **Abstract**

In a lymphangiogenesis inducing device (10), a wound application unit (14) is an edge (40) formed on an outer periphery (34) of a rod (12). The wound application unit (14) applies a wound (52) to a target site (50) by rotating the rod (12) about the central axis (22).

## Description

### Technical Field

The present invention relates to a lymphangiogenesis inducing device.

### Background Art

A lymphatic vessel is one of routes for recovering tissue fluid in a living body. When the lymphatic vessel is obstructed, tissue fluid stagnates. As a result, lymphedema accompanied by functional deterioration such as swelling and sensory paralysis occurs in the limbs such as arms and legs. Lymphedema is often developed by lymphadenectomy or radiation therapy performed as a part of cancer treatment such as breast cancer treatment.

Lymphedema is a disease that is difficult to eliminate completely once it has been developed. When becoming chronic, it is difficult to improve easily. If lymphedema is left as it is, the symptoms become more severe.

Currently, there is combined physical therapy as a treatment for lymphedema ("2018 edition, Lymphedema Clinical Practice Guideline", edited by The Japanese Lymphedema Society, pp. 81 to 83). The combined physical therapy is a treatment method in which a plurality of therapies is appropriately combined and performed on a site where the swelling of the body occurs according to the condition of the patient (Kotaro Suehiro, Ann Vasc Surg. 2020 Jan; 62: 258-262). Examples of the plurality of therapies include compression therapy in which an elastic dressing, an elastic bandage and the like are wrapped to apply a compression force, skin care, exercise under compression, massage and the like.

As a radical treatment method for lymphedema, there is lymphaticovenular anastomosis (LVA). Lymphaticovenular anastomosis is a procedure in which an anastomosis is performed so as to connect an obstructed lymphatic vessel and a vein (Takumi Yamamoto et al., "SCIP flap transplantation for lymphedema", PEPARS, No. 150, pp. 54 to 64, 2019).

### Summary of Invention

Complex physical therapy is a conservative therapy primarily aimed at slowing the progression of lymphedema. The symptoms do not improve. Therefore, daily self-care including pressure is required, and a burden is large for the patient.

Lymphaticovenular anastomosis is considered to be a radical therapy. However, lymphaticovenular anastomosis requires advanced plastic surgical techniques and there are limited opportunities to receive treatment. There is a case where veins and lymphatic vessels suitable for anastomosis are not found. There also is a case where thrombus occurs in the anastomosed blood vessel. Therefore, even when treatment is performed, a sufficient effect is not obtained in some cases. It is also said that the quality of the anastomosis affects the outcome of the treatment. Therefore, as for lymphaticovenular anastomosis, a unified procedure has not been determined. Therefore, in "2018 edition, Lymphedema Clinical Practice Guideline", pp. 81 to 83, it is only described that it is worth considering for patients who cannot undergo compression therapy.

Therefore, it is desired to perform radical treatment without requiring a difficult procedure.

An object of the present invention is to solve the above problem.
(1) An aspect of the present invention is a lymphangiogenesis inducing device including a rod to be inserted into a living tissue, and a wound application unit that is provided on the rod and applies a fine wound to a target site of the living tissue, in which the wound application unit is an edge formed on an outer periphery of the rod, and applies the wound to the target site by rotating the rod about a central axis.

According to the present invention, since the edge, which is the wound application unit, is formed on the outer periphery of the rod, the lymphangiogenesis inducing device can be easily configured. The wound can be easily applied to the target site by rotating the rod about the central axis. Moreover, the wound can be applied in a broad range with a small number of rotations by rotating the rod about the central axis. Furthermore, since the number of parts of the lymphangiogenesis inducing device is small, the cost can be reduced.

By configuring the lymphangiogenesis inducing device in this manner, it is possible to insert the rod into the living tissue and apply the fine wound to the target site without requiring a complicated procedure. By applying the fine wound to the target site, it is possible to promote the regeneration of the lymphatic vessel in the healing process of the wound.

(2) In the lymphangiogenesis inducing device according to (1) described above, the edge can be formed at an acute angle in a cross-sectional view as seen in an axial direction of the rod.

Since the edge is formed at the acute angle, when the rod is rotated about the central axis at the target site of the living tissue, the fine wound can be easily applied to the target site.

(3) The lymphangiogenesis inducing device according to (1) or (2) described above further includes an uneven portion formed on the outer periphery, in which
the edge may be a corner of the uneven portion.

Since the corner of the uneven portion serves as the edge by forming the uneven portion on the outer periphery, the edge can be easily formed. When the rod is rotated about the central axis at the target site of the living tissue, the fine wound can be easily applied to the target site.

(4) In the lymphangiogenesis inducing device according to (3) described above, the uneven portion may be a groove.

Since the uneven portion is the groove, the corner of the groove serves as the edge. As a result, the edge can be formed easily. When the rod is rotated about the central axis at the target site of the living tissue, the fine wound can be easily applied to the target site.

(5) In the lymphangiogenesis inducing device according to any one of (1) to (4) described above, the edge may extend in an axial direction of the rod on the outer periphery.

On the outer periphery, since the edge extends in the axial direction over an entire rod, when the rod is rotated about the central axis with respect to the target site of the living tissue, the fine wound can be easily applied in a broad range. Since it is sufficient to form the edge straight in the axial direction of the rod, the edge can be easily and simply formed.

(6) In the lymphangiogenesis inducing device according to (5) described above, the rod may include a distal end capable of puncturing the living tissue and a body continuous from the distal end, and the edge may be formed on the distal end and the body.

Since the edge is formed on the distal end and the body of the rod, when the distal end of the rod is inserted into the living tissue and the rod is displaced about the central axis with respect to the target site of the living tissue, the fine wound can be applied to the target site without damaging the skin of the living body. Since the rod includes the distal end capable of puncturing the living tissue, the rod can be used as the puncture device.

(7) In the lymphangiogenesis inducing device according to any one of (1) to (5) described above, the rod may include a distal end capable of puncturing the living tissue and a body continuous from the distal end, and
the edge may be formed not on the distal end but on the body.

Since the edge is formed on the body of the rod, when the distal end of the rod is inserted into the living tissue and the rod is rotated about the central axis with respect to the target site in a state in which the edge is located at the target site of the living tissue, the fine wound can be applied to the target site without damaging the skin of the living body. Since the rod includes the distal end capable of puncturing the living tissue, the rod can be used as the puncture device.

(8) In the lymphangiogenesis inducing device according to any one of (1) to (7) described above, the rod may be a hollow rod.

Since the rod is the hollow rod, it is possible to externally supply the chemical solution or the filamentous device to the target site through the rod.

(9) In the lymphangiogenesis inducing device according to any one of (1) to (7) described above, the rod may be a solid rod.

Since the rod is the solid rod, the rod becomes sturdy and suppressed from bending, and the lymphangiogenesis inducing device can be easily formed.

(10) In the lymphangiogenesis inducing device according to any one of (1) to (9) described above, a plurality of edges may be formed at an interval in a circumferential direction of the outer periphery.

A plurality of edges is formed at an interval in a circumferential direction of the outer periphery. As a result, when the rod is rotated about the central axis with respect to the target site of the living tissue, the fine wound can be applied to the target site only by rotating the same at a rotation angle smaller than that of one rotation (360°).

(11) In the lymphangiogenesis inducing device according to (10) described above, two edges may be formed on the outer periphery so as to face each other across the central axis.

Since the two edges are formed on the outer periphery so as to face each other across the central axis, only by rotating the rod about the central axis at the target site of the living tissue by 180°, the fine wound can be easily applied to the target site.

(12) The lymphangiogenesis inducing device according to any one of (1) to (11) described above further includes an uneven portion formed on the outer periphery so as to straddle a straight line extending in a radial direction through the central axis in a cross-sectional view as seen in an axial direction of the rod, in which the edge may be a corner of the uneven portion.

In the cross-sectional view as seen in the axial direction of the rod, the uneven portion is formed on the outer periphery so as to straddle the straight line extending in the radial direction through the central axis of the rod. As a result, the corner of the uneven portion serves as the edge, so that the edge can be easily formed. When the rod is rotated about the central axis at the target site of the living tissue, the fine wound can be easily applied to the target site.

According to the present invention, since the edge, which is the wound application unit, is formed on the outer periphery of the rod, the lymphangiogenesis inducing device can be easily configured. The wound can be easily applied to the target site by rotating the rod about the central axis. Moreover, the wound can be applied in a broad range with a small number of rotations by rotating the rod about the central axis. Furthermore, since the number of parts of the lymphangiogenesis inducing device is small, the cost can be reduced.

By configuring the lymphangiogenesis inducing device in this manner, it is possible to insert the rod into the living tissue and apply the fine wound to the target site without requiring a complicated procedure. By applying the fine wound to the target site, it is possible to promote the regeneration of the lymphatic vessel in the healing process of the wound.

### Brief Description of Drawings

[Fig. 1]

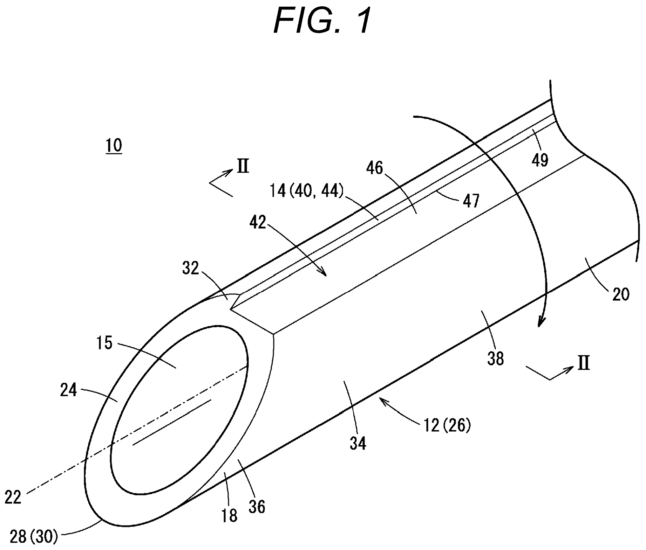

   Fig. 1 is a perspective view of a lymphangiogenesis inducing device according to the present embodiment.
[Fig. 2]

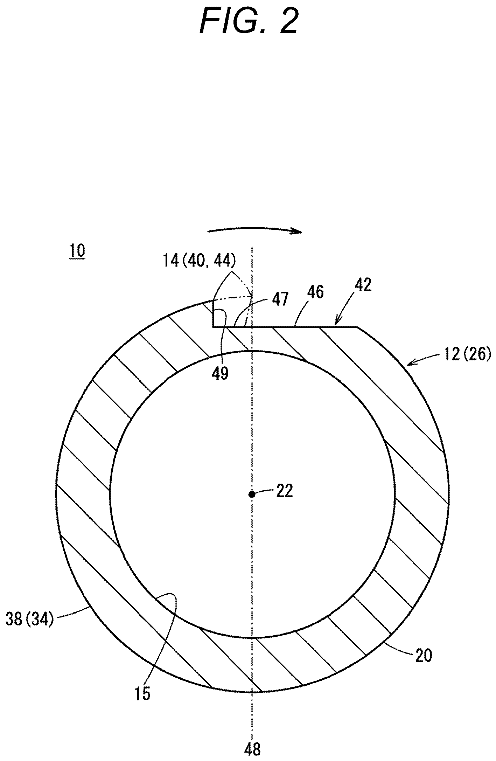

   Fig. 2 is a cross-sectional view taken along line II-II in Fig. 1.
[Fig. 3]

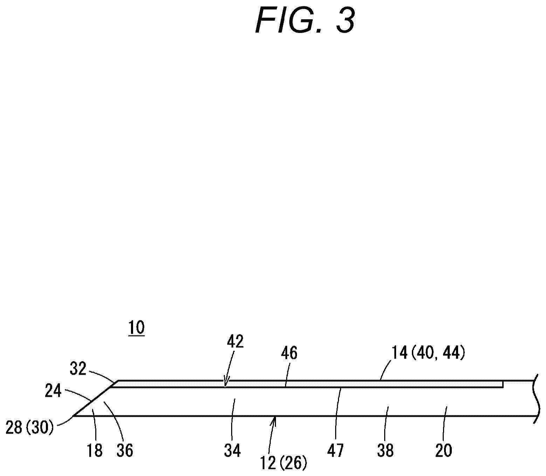

   Fig. 3 is a side view of the lymphangiogenesis inducing device in Fig. 1.
[Fig. 4]

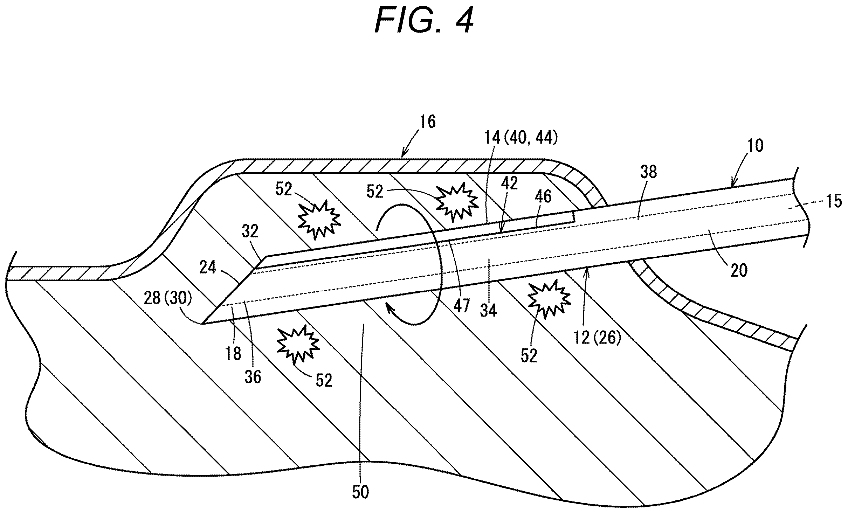

   Fig. 4 is an illustrative diagram illustrating a wound to a target site of a living tissue by the lymphangiogenesis inducing device of Fig. 1.
[Fig. 5]

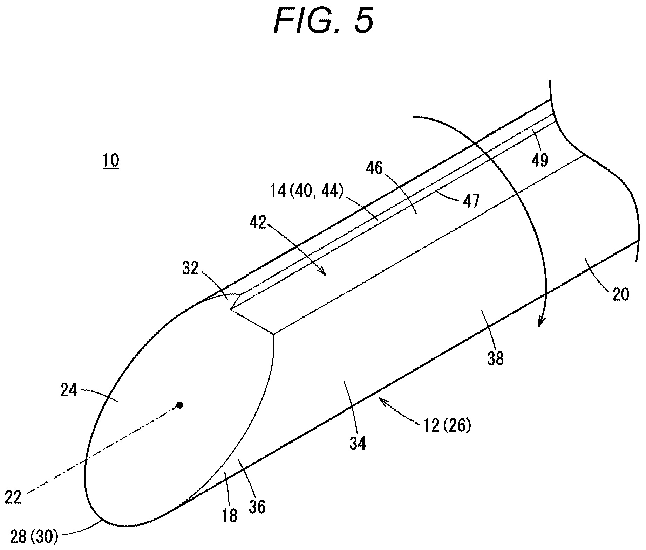

   Fig. 5 is a perspective view illustrating a case where the rod is solid.
[Fig. 6]

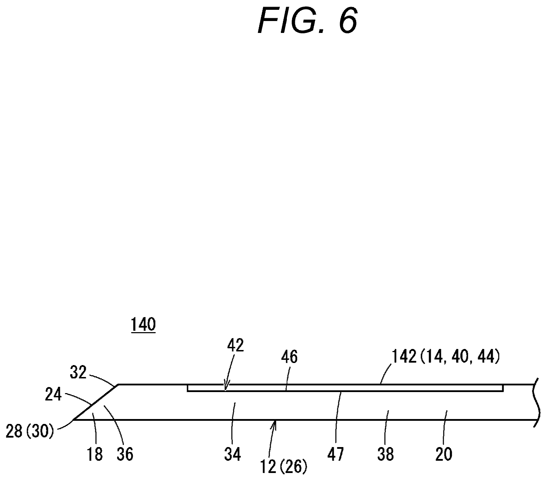

   Fig. 6 is a side view illustrating a first modification.
[Fig. 7]

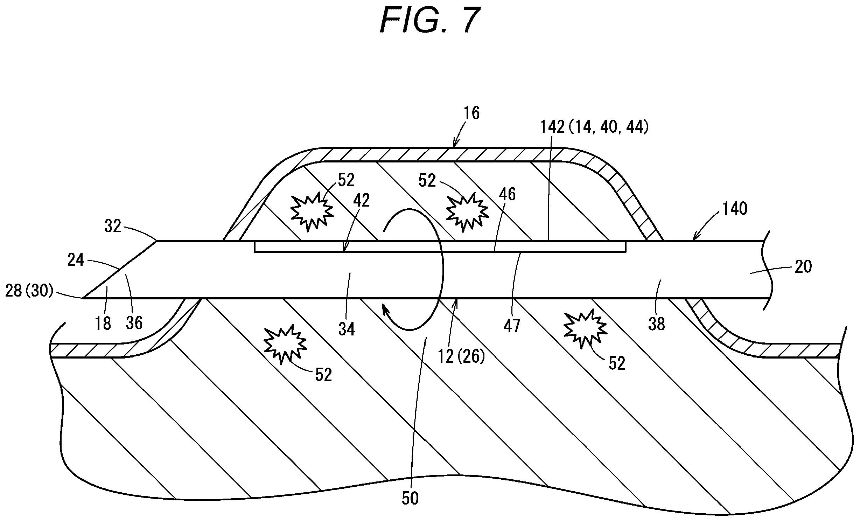

   Fig. 7 is an illustrative diagram illustrating a wound to a target site of a living tissue by the lymphangiogenesis inducing device of Fig. 6.
[Fig. 8]

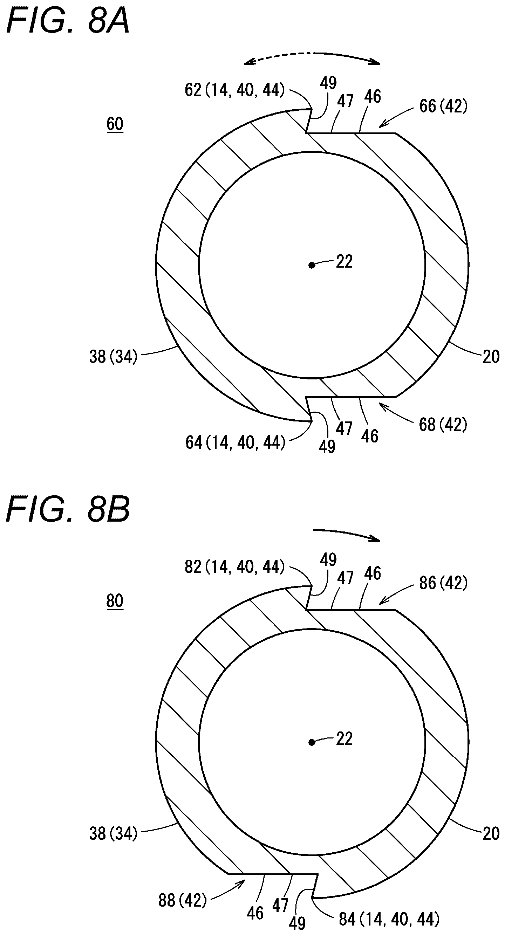

   Fig. 8A is a cross-sectional view illustrating a second modification, and Fig. 8B is a cross-sectional view illustrating a third modification.

### Description of Embodiments

Fig. 1 is a perspective view of a lymphangiogenesis inducing device 10 according to the present embodiment.

The lymphangiogenesis inducing device 10 includes a rod 12 and a wound application unit 14.

The rod 12 is a rod member made of metal. The rod 12 is a linear member. The rod 12 is a hollow rod. In the rod 12, a cavity hollow portion 15 is formed in an axial direction of the rod 12. The rod 12 has an outer diameter that allows advance inside a living tissue 16 (refer to Fig. 4). The rod 12 includes a distal end 18 and a body 20 continuous from the distal end 18.

The distal end 18 of the rod 12 has a sharp shape so as to be able to puncture the living tissue 16. Specifically, the distal end 18 of the rod 12 includes a blade surface 24 cut out obliquely with respect to a central axis 22 of the rod 12. The distal end 18 of the rod 12 can puncture the living tissue 16. Therefore, the rod 12 is a puncture device 26. The body 20 of the rod 12 extends in the axial direction along the central axis 22 of the rod 12.

The distal end 18 of the rod 12 is a portion from a most distal end 28 of the rod 12 (a needle tip 30 of the puncture device 26, which is the most distal end 28 of the blade surface 24) to a proximal end 32 of the blade surface 24. Therefore, the blade surface 24 is not formed on the body 20. An outer periphery 34 of the rod 12 includes an outer peripheral surface 36 of the distal end 18 and an outer peripheral surface 38 of the body 20.

The wound application unit 14 is formed on the outer periphery 34 of the rod 12. The wound application unit 14 is an edge 40 formed on the outer periphery 34 of the rod 12.

Specifically, an uneven portion 42 is formed on the outer periphery 34 of the rod 12. The uneven portion 42 extends in the axial direction of the rod 12. The uneven portion 42 reaches the blade surface 24. The uneven portion 42 is formed on the outer peripheral surface 36 of the distal end 18 and the outer peripheral surface 38 of the body 20. As illustrated in Fig. 3, the uneven portion 42 is formed on the distal end 18 and a part of the body 20. Alternatively, the uneven portion 42 may extend in the axial direction from the distal end 18 to a proximal end of the rod 12. The edge 40 is a corner 44 of the uneven portion 42.

In Figs. 1 and 2, the uneven portion 42 is formed by recessing a part of the outer periphery 34 of the rod 12 toward an inside of the rod 12. In Fig. 1, the uneven portion 42 includes a flat portion 46 and the corner 44.

The flat portion 46 is a portion recessed inward from the outer periphery 34 of the rod 12. That is, the flat portion 46 is a recessed portion of the uneven portion 42. The flat portion 46 is a plane orthogonal to a straight line 48 extending in a radial direction from the central axis 22. Specifically, as illustrated in Fig. 2, in a cross-sectional view as seen in the axial direction of the rod 12, the uneven portion 42 is formed on the outer periphery 34 of the rod 12 so as to straddle the straight line 48 extending in the radial direction through the central axis 22.

As illustrated in Fig. 1, the corner 44 is formed on the outer peripheral surface 38 of the body 20 and the outer peripheral surface 36 of the distal end 18. The corner 44 is a protruding portion of the uneven portion 42. The corner 44 is a projected portion (projection) that is continuous from the flat portion 46 and projecting radially outward from the flat portion 46. The corner 44 is formed at a connection between a linear portion 49 extending radially outward from the flat portion 46 and the outer periphery 34. Note that, as illustrated in Fig. 2, the linear portion 49 is parallel to the straight line 48.

A groove 47 is formed by the flat portion 46 and the linear portion 49. That is, the uneven portion 42 is the groove 47 formed on the outer periphery 34.

The corner 44 is formed at an acute angle in a cross-sectional view as seen in the axial direction of the rod 12. In the present embodiment, as indicated by a two-dot chain line, the corner 44 may be formed more sharply. As a result, the edge 40 can be formed into a shape having a more acute angle.

A method of using the lymphangiogenesis inducing device 10 according to the present embodiment will be described with reference to Fig. 4.

The user inserts the rod 12 into the living tissue 16 by puncturing the living tissue 16 with the distal end 18 of the rod 12. The user inserts the rod 12 into the living tissue 16 in the axial direction, thereby inserting the wound application unit 14 into a target site 50 in the living tissue 16. Next, the user rotates the rod 12 once (360° rotation) about the central axis 22 (refer to Figs. 1 and 2) when the wound application unit 14 is located at the target site 50. In this case, the rod 12 is rotated once in a rotational direction indicated by a solid line. When the rod 12 rotates, the edge 40 having the acute angle abuts the target site 50. As a result, a fine wound 52 can be applied from the edge 40 to the target site 50. After rotating the rod 12 once, the user pulls out the rod 12 from the living tissue 16 by retracting the rod 12 in the axial direction. In this manner, by applying the fine wound 52 to the target site 50, it is possible to promote regeneration of a lymphatic vessel in a healing process of the wound 52.

Note that, when the rod 12 is inserted into the living tissue 16, the user can externally supply a chemical solution or a filamentous device to the target site 50 through the hollow portion 15 of the rod 12.

An effect of the lymphangiogenesis inducing device 10 according to the present embodiment will be described.

As illustrated in Figs. 1 and 2, since the edge 40, which is the wound application unit 14, is formed on the outer periphery 34 of the rod 12, the lymphangiogenesis inducing device 10 can be easily configured. The wound 52 can be easily applied to the target site 50 by rotating the rod 12 about the central axis 22. Moreover, the wound 52 can be applied in a broad range with a small number of rotations by rotating the rod 12 about the central axis 22. Furthermore, since the number of parts of the lymphangiogenesis inducing device 10 is small, the cost can be reduced.

By configuring the lymphangiogenesis inducing device 10 in this manner, it is possible to insert the rod 12 into the living tissue 16 and apply the fine wound 52 to the target site 50 without requiring a complicated procedure. By applying the fine wound 52 to the target site 50, it is possible to promote the regeneration of the lymphatic vessel in the healing process of the wound 52.

Since the edge 40 is formed at the acute angle, when the rod 12 is rotated about the central axis 22 at the target site 50 of the living tissue 16, the fine wound 52 can be easily applied to the target site 50.

Since the corner 44 of the uneven portion 42 serves as the edge 40 by forming the uneven portion 42 on the outer periphery 34, the edge 40 can be easily formed. When the rod 12 is rotated about the central axis 22 at the target site 50 of the living tissue 16, the fine wound 52 can be easily applied to the target site 50.

Since the uneven portion 42 is the groove 47, the corner 44 of the groove 47 serves as the edge 40. As a result, the edge 40 can be formed easily. When the rod 12 is rotated about the central axis 22 at the target site 50 of the living tissue 16, the fine wound 52 can be easily applied to the target site 50.

On the outer periphery 34, since the edge 40 extends in the axial direction over an entire rod 12, when the rod 12 is rotated about the central axis 22 with respect to the target site 50 of the living tissue 16, the fine wound 52 can be easily applied in a broad range. Since it is sufficient to form the edge 40 straight in the axial direction of the rod 12, the edge 40 can be easily and simply formed.

Since the edge 40 (uneven portion 42) is formed on the distal end 18 and the body 20 of the rod 12, when the distal end 18 of the rod 12 is inserted into the living tissue 16 and the rod 12 is displaced about the central axis 22 with respect to the target site 50 of the living tissue 16, the fine wound 52 can be applied to the target site 50 without damaging the skin of the living body. Since the rod 12 includes the distal end 18 capable of puncturing the living tissue 16, the rod 12 can be used as the puncture device 26.

Note that, a length of the uneven portion 42 is preferably 1.0 cm to 15.0 cm, and more preferably 5.0 cm to 10.0 cm in a case where both application of the wound 52 and indwelling of the filamentous device are performed. This makes it possible to easily apply the wound 52 and indwell the filamentous device.

Since the rod 12 is the hollow rod, it is possible to externally supply the chemical solution or the filamentous device to the target site 50 through the rod 12.

As illustrated in Fig. 2, in a cross-sectional view as seen in the axial direction of the rod 12, the uneven portion 42 is formed on the outer periphery 34 so as to straddle the straight line 48 extending in the radial direction through the central axis 22 of the rod 12. As a result, the corner 44 of the uneven portion 42 serves as the edge 40, so that the edge 40 can be easily formed. When the rod 12 is rotated about the central axis 22 at the target site 50 of the living tissue 16, the fine wound 52 can be easily applied to the target site 50.

Note that, as illustrated in Fig. 5, the rod 12 may be a solid rod. Since the rod 12 is the solid rod, the rod 12 becomes sturdy and suppressed from bending, and the lymphangiogenesis inducing device 10 can be easily formed.

A shape of the rod 12 is not limited to the linear shape. The rod 12 may have a curved shape to some extent.

The blade surface 24 is not necessarily formed on the rod 12. The rod 12 is only required to be inserted into the living tissue 16. For example, at the time of surgery of the living tissue 16, since the living tissue 16 is incised, the rod 12 can be inserted into the incised living tissue 16.

A case of applying the wound 52 to the target site 50 by rotating the rod 12 once (360° rotation) about the central axis 22 has been described. In the present embodiment, it is only necessary to rotate the rod 12 at least once. Therefore, the rod 12 may be rotated by 360° or more.

Cutting of the blade surface 24 may be one-surface cutting or two-surface cutting. In the case of one-surface cutting, the edge 40 (uneven portion 42) may be formed on the blade surface 24. In the case of two-surface cutting, the rod 12 can easily puncture the living tissue 16.

The uneven portion 42 may communicate with the hollow portion 15. That is, the uneven portion 42 may be a hole. The uneven portion 42 may be a bottomed hole or depression.

Next, modifications (first to third modifications) of the present embodiment will be described with reference to Figs. 6 to 8B. Note that, in the first to third modifications, the same configurations as those of the lymphangiogenesis inducing device 10 according to the present embodiment (refer to Figs. 1 to 4) are denoted by the same reference numerals, and a detailed description thereof will be omitted.

Fig. 6 is a side view of a lymphangiogenesis inducing device 140 according to the first modification. Fig. 7 is an illustrative diagram illustrating the wound 52 to the target site 50 of the living tissue 16 by the lymphangiogenesis inducing device 140. In the first modification, an edge 142 is not formed on the distal end 18 of the rod 12, and the edge 142 is formed on the body 20. Specifically, the edge 142 is formed on a site away from the distal end 18 in the body 20 of the rod 12. The edge 142 is formed on a part of the body 20. Fig. 7 illustrates the wound 52 from the edge 142 to the target site 50 in the first modification.

In the first modification also, the user inserts the rod 12 into the living tissue 16 by puncturing the living tissue 16 with the distal end 18 of the rod 12. The user inserts the rod 12 into the living tissue 16 such that the edge 142 is located at the target site 50. As described above, the edge 142 is not formed on the distal end 18, but the edge 142 is formed on a part of the body 20. Therefore, the user advances the rod 12 into the living tissue 16 and exposes the distal end 18 of the rod 12 outside from the living tissue 16. That is, the distal end 18 of the rod 12 penetrates the living tissue 16. As a result, the edge 142 is located at the target site 50. In this case also, the user inserts the rod 12 in such a manner that the edge 142 is not located on the skin of the living tissue 16.

Next, the user rotates the rod 12 once in a rotational direction indicated by a solid line in Fig. 7. When the rod 12 rotates, the edge 142 having an acute angle abuts the target site 50, and the fine wound 52 is applied from the edge 142 to the target site 50. After rotating the rod 12 once, the user pulls out the rod 12 from the living tissue 16 by retracting the rod 12 in the axial direction. Therefore, in the first modification also, by applying the fine wound 52 to the target site 50, it is possible to promote regeneration of a lymphatic vessel in a healing process of the wound 52.

An effect of the lymphangiogenesis inducing device 140 according to the first modification will be described.

In the first modification also, in addition to the effect of the present embodiment, the edge 142 is formed on the body 20 of the rod 12. Therefore, when the distal end 18 of the rod 12 is inserted into the living tissue 16 and the rod 12 is rotated about the central axis 22 with respect to the target site 50 in a state in which the edge 142 is located at the target site 50 of the living tissue 16, the fine wound 52 can be applied to the target site 50 without damaging the skin of the living body. Also in a case where the distal end 18 of the rod 12 penetrates the living tissue 16, the above-described effect can be easily obtained by locating the edge 142 at the target site 50 of the living tissue 16.

Note that, in the first modification, the length of the uneven portion 42 is preferably 1.0 cm to 15.0 cm, and more preferably 5.0 cm to 10.0 cm in a case where both application of the wound 52 and indwelling of the filamentous device are performed. This makes it possible to easily apply the wound 52 and indwell the filamentous device.

In the first modification, the length from the most distal end 28 to the uneven portion 42 in the axial direction and the length from the proximal end of the rod 12 to the uneven portion 42 in the axial direction are preferably 0.5 cm to 13.0 cm, and more preferably 1.0 cm to 8.0 cm. As a result, the edge 142 can be located at the target site 50 easily. The user can easily operate the rod 12.

Fig. 8A is a cross-sectional view of a lymphangiogenesis inducing device 60 according to the second modification. In the second modification, a plurality of edges 62 and 64 is formed at an interval in a circumferential direction of the outer periphery 34 of the rod 12. In Fig. 8A, as an example, two edges 62 and 64 are formed on the outer periphery 34 of the rod 12 so as to face each other across the central axis 22. That is, two uneven portions 66 and 68 are formed at an interval of 180°, and corners 44 of the two uneven portions 66 and 68 are formed as two edges 62 and 64. In the second modification, the two edges 62 and 64 face each other in the circumferential direction. That is, an upper edge 62 of the two edges 62 and 64 is an edge having an acute angle in the rotational direction of a solid line (a clockwise direction in Fig. 8A). The lower edge 64 is an edge having an acute angle in the rotational direction of a broken line (counterclockwise direction in Fig. 8A).

In the second modification, the user rotates the rod 12 once in the rotational direction of the solid line or the rotational direction of the broken line in Fig. 8A about the central axis 22 when the wound application unit 14 is located at the target site 50 (refer to Figs. 4 and 7). When the rod 12 is rotated in the rotational direction of the solid line, the upper edge 62 abuts the target site 50, and the fine wound 52 is applied from the edge 62 to the target site 50. When the rod 12 is rotated in the rotational direction of the broken line, the lower edge 64 abuts the target site 50, and the fine wound 52 is applied from the edge 64 to the target site 50. Therefore, in the second modification, the fine wound 52 can be applied from the edges 62 and 64 to the target site 50 regardless of the direction in which the rod 12 is rotated about the central axis 22.

An effect of the lymphangiogenesis inducing device 60 according to the second modification will be described.

In the second modification, in addition to the configuration of the present embodiment, a plurality of edges 62 and 64 is formed at an interval in the circumferential direction of the outer periphery 34. As a result, when the rod 12 is rotated about the central axis 22 with respect to the target site 50 of the living tissue 16, the fine wound 52 can be applied to the target site 50 only by rotating the same at a rotation angle smaller than that of one rotation (360°).

Since the two edges 62 and 64 are formed on the outer periphery 34 so as to face each other across the central axis 22, only by rotating the rod 12 about the central axis 22 at the target site 50 of the living tissue 16 by 180°, the fine wound 52 can be easily applied to the target site 50.

Fig. 8B is a cross-sectional view of a lymphangiogenesis inducing device 80 according to the third modification. In the third modification also, two edges 82 and 84 are formed on the outer periphery 34 of the rod 12 so as to face each other across the central axis 22. In the third modification, the two edges 82 and 84 are edges having an acute angle in the rotational direction of the solid line. That is, also in the third modification, the two uneven portions 86 and 88 are formed on the outer periphery 34 of the rod 12 so as to face each other across the central axis 22, and the corners 44 of the two uneven portions 86 and 88 are formed as the two edges 82 and 84.

In the third modification, the user rotates the rod 12 by 180° (half-rotation) in the rotational direction of the solid line about the central axis 22 when the wound application unit 14 is located at the target site 50 (refer to Figs. 4 and 7). As a result, each of the two edges 82 and 84 abuts the target site 50, and the fine wound 52 is applied from the edges 82 and 84 to the target site 50. Therefore, in the third modification, the fine wound 52 can be applied from the edges 82 and 84 to the target site 50 by rotating the rod 12 at the rotation angle smaller than one rotation.

In the lymphangiogenesis inducing device 80 according to the third modification, it is possible to obtain an effect similar to that of the second modification.

Note that, in the second modification and the third modification, three or more edges 62, 64, 82, and 84 may be formed on the outer periphery 34. As a result, the fine wound 52 can be applied to the target site 50 only by rotating the rod 12 at a rotation angle further smaller than the rotation angle of one rotation (360°).

Note that, the present invention is not limited to the above disclosure, and various configurations can be adopted without departing from the gist of the present invention.

## Claims

1. A lymphangiogenesis inducing device comprising:
a rod to be inserted into a living tissue; and
a wound application unit that is provided on the rod and applies a fine wound to a target site of the living tissue, wherein
the wound application unit is an edge formed on an outer periphery of the rod, and applies the wound to the target site by rotating the rod around a central axis.

2. The lymphangiogenesis inducing device according to claim 1, wherein
the edge is formed at an acute angle in a cross-sectional view as seen in an axial direction of the rod.

3. The lymphangiogenesis inducing device according to claim 1, further comprising:
an uneven portion formed on the outer periphery, wherein
the edge is a corner of the uneven portion.

4. The lymphangiogenesis inducing device according to claim 3, wherein
the uneven portion is a groove.

5. The lymphangiogenesis inducing device according to claim 1, wherein
the edge extends in an axial direction of the rod on the outer periphery.

6. The lymphangiogenesis inducing device according to claim 5, wherein
the rod includes a distal end capable of puncturing the living tissue and a body continuous from the distal end, and
the edge is formed on the distal end and the body.

7. The lymphangiogenesis inducing device according to claim 1, wherein
the rod includes a distal end capable of puncturing the living tissue and a body continuous from the distal end, and
the edge is formed not on the distal end but on the body.

8. The lymphangiogenesis inducing device according to claim 1, wherein
the rod is a hollow rod.

9. The lymphangiogenesis inducing device according to claim 1, wherein
the rod is a solid rod.

10. The lymphangiogenesis inducing device according to claim 1, wherein
a plurality of edges is formed at an interval in a circumferential direction of the outer periphery.

11. The lymphangiogenesis inducing device according to claim 10, wherein
two edges are formed on the outer periphery so as to face each other across the central axis.

12. The lymphangiogenesis inducing device according to claim 1, further comprising:
an uneven portion formed on the outer periphery so as to straddle a straight line extending in a radial direction through the central axis in a cross-sectional view as seen in an axial direction of the rod, wherein
the edge is a corner of the uneven portion.
